(19) Europäisches Patentamt / European Patent Office / Office européen des brevets

(11) EP 4 659 734 A1

(12) EUROPEAN PATENT APPLICATION
published in accordance with Art. 153(4) EPC

(43) Date of publication:
**10.12.2025 Bulletin 2025/50**

(21) Application number: **24750201.6**

(22) Date of filing: **29.01.2024**

(51) International Patent Classification (IPC):
***A61K 8/49*** *(2006.01)* ***A61K 8/19*** *(2006.01)*
***A61K 8/41*** *(2006.01)* ***A61Q 5/10*** *(2006.01)*

(52) Cooperative Patent Classification (CPC):
**A61K 8/19; A61K 8/41; A61K 8/49; A61Q 5/10**

(86) International application number:
**PCT/JP2024/002633**

(87) International publication number:
**WO 2024/162252 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.01.2023 JP 2023011706**

(71) Applicant: **Kao Corporation**
**Chuo-ku**
**Tokyo 103-8210 (JP)**

(72) Inventors:
- **SHIRATSUKI Ko**
  **Tokyo 131-8501 (JP)**
- **NAKAMURA Takahito**
  **Tokyo 131-8501 (JP)**

(74) Representative: **Hoffmann Eitle**
**Patent- und Rechtsanwälte PartmbB**
**Arabellastraße 30**
**81925 München (DE)**

# (54) METHOD FOR DYEING HAIR

(57) A method for dyeing hair, comprising the following step (I) and (II) in order:
(I) mixing an A part with a B part before use to prepare a hair dye composition, the A part comprising the following component (A1) and component (A2): (A1) a compound of the following formula (1) or a salt thereof wherein the broken line represents presence or absence of a $\pi$ bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and (A2) an alkali agent, the B part comprising the following component (B1) and component (B2): (B1) an oxidation dye other than the component (A1), and (B2) an alkali agent; and
(II) applying the hair dye composition to hair, wherein a mix ratio of the A part and the B part in the step (I) is in a predetermined ratio, and the method for dyeing hair comprises no step of applying an oxidizing agent to hair.

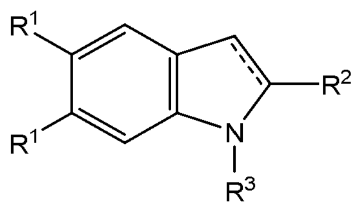

(1)

## Description

Field of the Invention

The present invention relates to a method for dyeing hair.

Background of the Invention

A technique of dyeing hair without use of an oxidizing agent such as hydrogen peroxide is demanded from the viewpoint of suppressing damage on hair.

An air oxidation hair dye composition is known which employs 5,6-dihydroxyindole, 5,6-dihydroxyindoline, or a derivative thereof (hereinafter, these are also collectively referred to as merely a "dihydroxyindole derivative"), which is a melanin precursor, as a coloring component for a gray hair dye. The above melanin precursor is a component which is converted to a melanin pigment by oxidative polymerization, and is susceptible to air oxidation to polymerization. Hence, the melanin precursor causes less damage on hair when used in a hair dye composition, and is also convenient as a coloring component.

Since hair dyed with the melanin precursor usually assumes gray or black color, a dyeing method only with the melanin precursor as a coloring component is exceedingly limited in terms of ranges of color expression. Accordingly, a hair dye agent and a hair dyeing method using a dihydroxyindole derivative and an oxidation dye in combination have been under investigation.

For example, JP-A-hei-3-58917 (JP-A-1991-58917, Patent Literature 1) discloses a method for dyeing keratinous fiber, including: a first step of applying a composition (A) containing a predetermined indole dye or an addition salt with an inorganic acid or an organic acid, an alkali metal salt, an alkaline earth metal salt, or a salt of corresponding amine thereof to keratinous fiber, particularly, human keratinous fiber, in a medium suitable for dyeing containing no oxidizing agent; and a second step of applying a composition (B) containing a predetermined oxidation dye to the keratinous fiber in a medium suitable for dyeing, wherein the composition (B) is mixed, if necessary, with hydrogen peroxide upon application.

JP-A-hei-1-252673 (JP-A-1989-252673, Patent Literature 2), JP-A-hei-3-58916 (JP-A-1991-58916, Patent Literature 3), JP-A-hei-4-295414 (JP-A-1992-295414, Patent Literature 4), Chinese Patent Publication No. 114177114 (Patent Literature 5), and JP-A-2001-511436 (Patent Literature 6) disclose methods for dyeing hair using one-part hair dyes in which a dihydroxyindole derivative is blended with various oxidation dyes.

Summary of the Invention

The present invention relates to the following.

[1] A method for dyeing hair, comprising the following steps (I) and (II) in order:

(I) mixing an A part with a B part before use to prepare a hair dye composition,

the A part comprising the following component (A1) and component (A2):

(A1) a compound of the following formula (1) or a salt thereof:

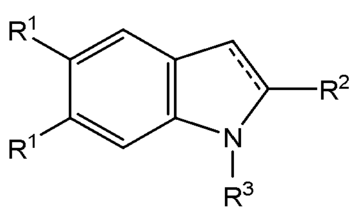

(1)

wherein the broken line represents presence or absence of a π bond; $R^1$ represents a hydroxy group or an acetoxy group; $R^2$ represents a hydrogen atom or -COOR, wherein R represents a hydrogen atom, a methyl group, or an ethyl group; and $R^3$ represents a hydrogen atom, an acetyl group, a methyl group, or an ethyl group, and

(A2) an alkali agent,

the B part comprising the following component (B1) and component (B2):

(B1) an oxidation dye other than the component (A1), and
(B2) an alkali agent; and

(II): applying the hair dye composition to hair, wherein

a mix ratio of the A part and the B part in the step (I) is such that a molar ratio of the component (A1) to the component (B1), [(A1)/(B1)], is from 10/90 to 70/30, and
the method for dyeing hair comprises no step of applying an oxidizing agent to hair.

[2] A kit for dyeing hair, comprising:

an A part comprising the following component (A1) and component (A2):

(A1) a compound of the following formula (1) or a salt thereof:

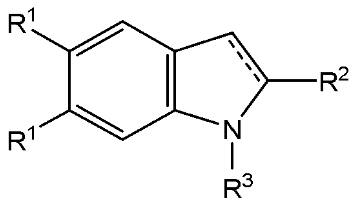
(1)

wherein the broken line represents presence or absence of a $\pi$ bond; $R^1$ represents a hydroxy group or an acetoxy group; $R^2$ represents a hydrogen atom or -COOR, wherein R represents a hydrogen atom, a methyl group, or an ethyl group; and $R^3$ represents a hydrogen atom, an acetyl group, a methyl group, or an ethyl group, and
(A2) an alkali agent; and

a B part comprising the following component (B1) and component (B2):

(B1) an oxidation dye other than the component (A1), and
(B2) an alkali agent,

the kit comprising no preparation containing an oxidizing agent.

Detailed Description of the Invention

The method disclosed in Patent Literature 1 includes: a first step of applying a composition containing a predetermined indole dye or a salt thereof to keratinous fiber; and a second step of applying a composition containing a predetermined oxidation dye to the keratinous fiber. Therefore, at least two steps are essential. Hence, the dyeing process is time-consuming. In addition, an intended color is difficult to attain due to difference in bath ratio or step interval. Furthermore, the second stage substantially requires hydrogen peroxide and might thus cause hair damage as mentioned above.
All the methods disclosed in Patent Literatures 2 to 6 are methods using a one-part hair dye and therefore require providing a plurality of one-part hair dyes and blending them upon application, for enhancing hair dyeability and performing expression of various colors. Thus, the operation is complicated.
The present invention relates to a method for dyeing hair using a melanin precursor, wherein the method for dyeing hair causes less hair damage ascribable to dyeing, has favorable hair dyeability, and can easily express various colors.
The present inventors found that the above problems can be solved by a method for dyeing hair, including the steps of: mixing an A part containing a melanin precursor and an alkali agent with a B part containing an oxidation dye and an alkali agent at a predetermined ratio before use to prepare a hair dye composition; and applying the hair dye composition to hair, the method having no step of applying an oxidizing agent to hair.
The present invention provides a method for dyeing hair using a melanin precursor, wherein the method for dyeing hair causes less hair damage ascribable to dyeing, has favorable hair dyeability, and can easily express various colors.

[Definition]

In the present specification, the term "favorable hair dyeability" means a high degree of hair dyeing. The "high degree of hair dyeing" means a large color difference (ΔE) of hair between before and after dyeing. Specifically, the ΔE can be evaluated in accordance with a method described in Examples.

In the present specification, the phrase "can easily express various colors" means that colors can be easily changed by merely mixing the A part and the B part, which are materials of a hair dye composition, at varied ratios, without blending multiple hair dyes. The variety of the color expression can be evaluated in accordance with a method hereinafter using a difference in chroma C value (ΔC) between a treatment with each of the A part and the B part alone and a treatment with a mixture thereof.

[Method for dyeing hair]

The method for dyeing hair according to the present invention (hereinafter, also referred to as the "hair dyeing method of the present invention" or simply the "method of the present invention") is a method for dyeing hair, comprising the following step (I) and (II) in order:

(I)mixing an A part with a B part before use to prepare a hair dye composition,

the A part comprising the following component (A1) and component (A2):

(A1) a compound of the following formula (1) or a salt thereof:

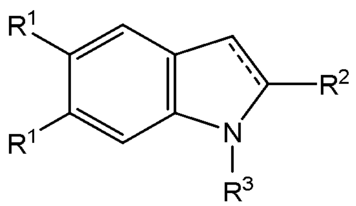

(1)

wherein the broken line represents presence or absence of a π bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and

(A2) an alkali agent

the B part comprising the following component (B1) and component (B2):

(B1) an oxidation dye other than the component (A1), and

(B2) an alkali agent; and

(II) applying the hair dye composition to hair, wherein

a mix ratio of the A part and the B part in the step (I) is such that a molar ratio of the component (A1) to the component (B1), [(A1)/(B1)], is from 10/90 to 70/30, and

the method for dyeing hair comprises no step of applying an oxidizing agent to hair.

In the present specification, expression of various colors is easily performed by merely mixing the A part and the B part, which are materials of a hair dye composition, at varying ratios, as described above, and does not require blending a plurality of hair dyes. On the other hand, a known usual two-part hair dye is composed of one first part (a dye and an alkali agent) and a second part (hydrogen peroxide) and can represent only one color because these parts are mixed at one fixed ratio. Even if the ratio is changed, only the darkness of dyed hair is changed. This type of hair dye requires blending the hair dye with another first part upon application, for expressing diverse colors.

The hair dyeing method of the present invention, by having the configuration described above, causes less hair damage ascribable to dyeing, has favorable hair dyeability, and can easily express various colors even if the melanin precursor component (A1) is used as a coloring component.

The present invention exerts the effects described above, presumably because:

the method of the present invention, unlike a method using a one-part hair dye composition, employs the A part and the B part mixed before use, and can therefore easily achieve a desired color by changing a mix ratio of the A part and the B part.

The mix ratio of the A part and the B part is adjusted such that a molar ratio [(A1)/(B1)] is in a determined range. This enables various colors to be expressed even if the component (A1) which usually exhibits gray or black color is used as a coloring component. Furthermore, in a hair dyeing process, sufficient hair dyeability is obtained by air oxidation without the use of an oxidizing agent. Therefore, hair damage can presumably also be suppressed.

<Step (I)>

The step (I) involves mixing an A part with a B part before use to prepare a hair dye composition;

the A part comprising the following component (A1) and component (A2):

(A1) a compound of the following formula (1) or a salt thereof:

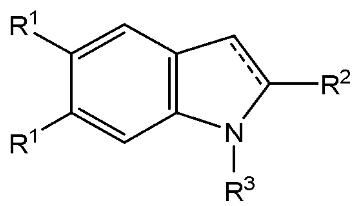

(1)

wherein the broken line represents presence or absence of a π bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and
(A2) an alkali agent,

the B part comprising the following component (B1) and component (B2):

(B1) an oxidation dye other than the component (A1), and
(B2) an alkali agent.

(A part)

The A part for use in the step (I) contains the following component (A1) and component (A2):

(A1) a compound of the following formula (1) or a salt thereof:

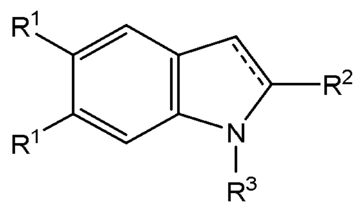

(1)

wherein the broken line represents presence or absence of a π bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and
(A2) an alkali agent.

[Component (A1): compound of formula (1) or salt thereof]

The A part contains a component (A1) which is a compound of the formula (1) or a salt thereof. The component (A1) is a melanin precursor which is polymerized by air oxidation and thereby converted to a melanin pigment, and acts as a hair dye.

The compound of the formula (1) is an indole derivative or an indoline derivative, or a salt thereof. In the present invention, one or more thereof can be used in combination.

The component (A1) is more preferably an indole derivative (i.e., a $\pi$ bond is present in a moiety of the broken line in the formula (1)) from the viewpoint of improvement in hair dyeability and from the viewpoint of easiness in expression of various colors.

In the formula (1), $R^1$ is preferably a hydroxy group, and $R^2$ is preferably a hydrogen atom or -COOR (wherein R is a hydrogen atom, a methyl group, or an ethyl group), more preferably a hydrogen atom or -COOH, from the viewpoint of availability of the component (A1) and improvement in hair dyeability and from the viewpoint of easiness in expression of various colors. $R^3$ is preferably a hydrogen atom.

Examples of the compound of the formula (1) include 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-hydroxyindole, 5-acetoxy-6-hydroxyindole-2-carboxylic acid, 5,6-dihydroxyindoline, 5,6-dihydroxyindoline-2-carboxylic acid, methyl 5,6-dihydroxyindoline-2-carboxylate, ethyl 5,6-dihydroxyindoline-2-carboxylate, N-methyl-5,6-dihydroxyindoline, N-methyl-5,6-dihydroxyindoline-2-carboxylic acid, N-ethyl-5,6-dihydroxyindoline, N-ethyl-5,6-dihydroxyindoline-2-carboxylic acid, N-acetyl-5,6-dihydroxyindoline, N-acetyl-5,6-dihydroxyindoline-2-carboxylic acid, 5-acetoxy-6-hydroxyindoline, and 5-acetoxy-6-hydroxyindoline-2-carboxylic acid, one or more of which can be used.

Examples of the salt of the compound of the formula (1) include hydrochloride, hydrobromide, sulfate, phosphate, acetate, propionate, lactate, and citrate of any of the compounds. Hydrobromide is preferred from the viewpoint of availability and improvement in hair dyeability and from the viewpoint of easiness in expression of various colors.

When $R^2$ in the formula (1) is -COOH, examples of the salt of the compound of the formula (1) include carboxylate thereof (wherein $R^2$ is $-COO^-X^+$ ($X^+$ is a cation such as an alkali metal ion (e.g., $Na^+$ and $K^+$), an alkaline earth metal ion (e.g., $Ca^+$ and $Mg^+$), or an ammonium ion)).

The component (A1) is preferably one or more members selected from the group consisting of 5,6-dihydroxyindole, 5,6-dihydroxyindole-2-carboxylic acid, 5,6-dihydroxyindoline, 5,6-dihydroxyindoline-2-carboxylic acid, and a salt thereof, is more preferably one or more members selected from the group consisting of 5,6-dihydroxyindole, 5,6-dihydroxyindole-2-carboxylic acid, and 5,6-dihydroxyindoline hydrobromide, is further more preferably one or more members selected from the group consisting of 5,6-dihydroxyindole and 5,6-dihydroxyindole-2-carboxylic acid, even more preferably contains 5,6-dihydroxyindole, and even more preferably contains 5,6-dihydroxyindole and 5,6-dihydroxyindole-2-carboxylic acid, from the viewpoint of improvement in hair dyeability and from the viewpoint of easiness in expression of various colors.

When the component (A1) contains 5,6-dihydroxyindole and 5,6-dihydroxyindole-2-carboxylic acid, a molar ratio of the 5,6-dihydroxyindole and the 5,6-dihydroxyindole-2-carboxylic acid is preferably in the range of from 50 : 50 to 99 : 1, more preferably in the range of from 80 : 20 to 99 : 1, further more preferably in the range of from 85 : 15 to 95 : 5, from the viewpoint of improvement in hair dyeability and from the viewpoint of easiness in expression of various colors.

When the component (A1) contains 5,6-dihydroxyindole and 5,6-dihydroxyindole-2-carboxylic acid, the molar ratio of the 5,6-dihydroxyindole and the 5,6-dihydroxyindole-2-carboxylic acid in the component (A1) can be quantified by reverse-phase HPLC.

A content of the component (A1) in the A part is preferably 0.02% by mass or more, more preferably 0.05% by mass or more, further more preferably 0.1% by mass or more, from the viewpoint of improvement in hair dyeability and from the viewpoint of easiness in expression of various colors, and preferably 5.0% by mass or less, more preferably 3.0% by mass or less, further more preferably 2.0% by mass or less, even more preferably 1% by mass or less, even more preferably 0.8% by mass or less, even more preferably 0.5% by mass or less, from the viewpoint of flexibility in formulation.

[Component (A2): alkali agent]

The component (A2) has the action of improving hair dyeability through the action of swelling hair so as to open cuticle and allowing a coloring component such as the component (A1) to penetrate the inside of the hair.

Any alkali agent for use in a usual hair dye composition can be used without particular limitations as the component (A2), which may be any of inorganic alkali agents and organic alkali agents. The component (A2) is preferably ammonia or an organic alkali agent from the viewpoint of improvement in hair dyeability.

Examples of the alkali agent which is preferred as the component (A2) include: ammonia; alkanolamine such as mono-, di-, or trimethanolamine, mono-, di-, or triethanolamine, trishydroxymethylaminomethane, and 2-amino-2-methyl-1-propanol; alkylamine such as methylamine, dimethylamine, ethylamine, diethylamine, N-methylethylamine, propylamine, and butylamine; and aralkylamine such as benzylamine, one or more of which can be used. The alkanolamine, the alkylamine, or the aralkylamine has preferably 1 or more and 10 or less, more preferably 1 or more and 8 or less carbon atoms from the viewpoint of water solubility.

Among those described above, the component (A2) preferably contains one or more members selected from the group consisting of ammonia, alkanolamine, alkylamine, and aralkylamine, more preferably one or more members

selected from the group consisting of ammonia and alkanolamine, and further more preferably alkanolamine, from the viewpoint of improvement in hair dyeability and from the viewpoint of easiness in expression of various colors. The component (A2), as the alkanolamine, preferably contains one or more members selected from the group consisting of monoethanolamine, trishydroxymethylaminomethane, and 2-amino-2-methyl-1-propanol, more preferably one or more members selected from the group consisting of monoethanolamine and 2-amino-2-methyl-1-propanol, and further more preferably monoethanolamine, from the viewpoint of improvement in hair dyeability, and from the viewpoint of easiness in expression of various colors.

A content of the alkanolamine in the component (A2) is preferably 70% by mass or more, more preferably 80% by mass or more, further more preferably 90% by mass or more, and 100% by mass or less, from the viewpoint of improvement in hair dyeability and from the viewpoint of easiness in expression of various colors.

A content of the component (A2) in the A part is preferably 0.01% by mass or more, more preferably 0.1% by mass or more, further more preferably 0.5% by mass or more, even more preferably 1.0% by mass or more, even more preferably 2.0% by mass or more, from the viewpoint of allowing the component (A1) to penetrate the inside of hair while improving hair dyeability by accelerating the polymerization reaction of the component (A1) in the hair, and preferably 10% by mass or less, more preferably 7.5% by mass or less, further more preferably 5.0% by mass or less, from the viewpoint of suppressing hair damage.

[Component (A3): antioxidant]

The A part preferably further contains an antioxidant as a component (A3) from the viewpoint of suppressing the oxidative polymerization of the component (A1) outside hair.

Examples of the antioxidant include sulfurous acid, pyrosulfurous acid, hydrogen sulfite, sulfur dioxide, L-ascorbic acid, thioglycolic acid, L-cysteine, N-acetyl-L-cysteine, and a salt thereof, and a derivative thereof. Examples of the salt include alkali metals such as sodium and potassium.

Among those described above, the antioxidant is preferably one or more members selected from the group consisting of sulfite, pyrosulfite, hydrosulfite, sulfur dioxide, L-ascorbic acid, and L-ascorbate, more preferably one or more members selected from the group consisting of sodium sulfite and sodium L-ascorbate, further more preferably sodium sulfite, from the viewpoint of the effect of suppressing the oxidative polymerization of the component (A1) outside hair.

When the A part contains the antioxidant (A3), a content of the component (A3) in the A part is preferably 0.01% by mass or more, more preferably 0.05% by mass or more, further more preferably 0.1% by mass or more, even more preferably 0.2% by mass or more, and preferably 10% by mass or less, more preferably 5.0% by mass or less, further more preferably 3.0% by mass or less, even more preferably 2.0% by mass or less, even more preferably 1.0% by mass or less, even more preferably 0.5% by mass or less, from the viewpoint of improvement in the effect of suppressing the oxidative polymerization of the component (A1) outside hair.

When the A part contains the antioxidant(A3), a mass ratio of the component (A3) to the component (A1), [(A3)/(A1)], in the A part is preferably 0.05 or more, more preferably 0.1 or more, further more preferably 0.2 or more, even more preferably 0.3 or more, even more preferably 0.5 or more, even more preferably 0.8 or more, even more preferably 1.0 or more, even more preferably 1.2 or more, from the viewpoint of the effect of suppressing the oxidative polymerization of the component (A1) outside hair. The mass ratio is preferably 10 or less, more preferably 5.0 or less, further more preferably 3.0 or less, even more preferably 2.0 or less, from the viewpoint of improvement in hair dyeability.

[pH adjuster]

The A part preferably further contains a pH adjuster from the viewpoint of adjusting pH to a desired range described hereinafter, accelerating the polymerization reaction within hair of the component (A1), and improving hair dyeability.

Since the A part contains the alkali agent as the component (A2), the pH adjuster for use in the A part is preferably a protonating agent. The protonating agent may be any of monobasic acids and polybasic acids or may be any of organic acids (having 1 or more and 8 or less carbon atoms, except for ascorbic acid) and inorganic acids.

Examples of the protonating agent include one or more members selected from the group consisting of hydrochloric acid, sulfuric acid, phosphoric acid, formic acid, acetic acid, and citric acid. One or more members selected from the group consisting of phosphoric acid and citric acid is preferred from the viewpoint of adjusting pH of the A part to a range described hereinafter.

When the A part contains the pH adjuster, a content of the pH adjuster in the A part is preferably 0.05% by mass or more, more preferably 0.1% by mass or more, further more preferably 0.2% by mass or more, and preferably 5.0% by mass or less, more preferably 4.0% by mass or less, further more preferably 3.5% by mass or less, even more preferably 2.5% by mass or less, even more preferably 1.5% by mass or less, from the viewpoint of adjusting pH of the A part to a range described hereinafter.

[Aqueous medium]

The A part usually further contains an aqueous medium. Examples of the aqueous medium include: water; lower alcohols such as ethanol and isopropyl alcohol; and low-molecular diol and triol having 6 or less carbon atoms, such as 1,3-butylene glycol, glycerin, ethylene glycol, and propylene glycol. The aqueous medium is preferably one or more members selected from the group consisting of water and ethanol, more preferably water, from the viewpoint of the solubility of the component (A1) and the component (A2), from the viewpoint of affinity for hair, and from the viewpoint of suppressing hair damage.

A content of the aqueous medium in the A part is usually in the range of from 1 to 99% by mass and can be appropriately selected in accordance with a formulation of the A part, and the like. The content of the aqueous medium in the A part is preferably 50% by mass or more, more preferably 70% by mass or more, further more preferably 80% by mass or more, from the viewpoint of easiness in mixing with the B part, and preferably 98% by mass or less from the viewpoint of easiness in expression of various colors.

The content of the aqueous medium in the A part may be a balance for the component (A1), the component (A2), the antioxidant, and the pH adjuster.

[Other components]

The A part may appropriately contain, in addition to the components described above, components which are conventionally used in hair dye compositions, without impairing the object of the present invention. Examples of the components include surfactants, viscosity adjusters, silicone, aromatic alcohols, coloring components other than the component (A1), polymers, oil agents, anti-dandruff agents, vitamin preparations, germicides, anti-inflammatory agents, antiseptics, chelating agents, humectants, pearlescent agents, a ceramide, fragrances, and ultraviolet absorbers.

The A part may contain a coloring component other than the component (A1). However, according to the hair dyeing method of the present invention, the A part may substantially contain no coloring component other than the component (A1). The phrase "substantially contain no coloring component other than the component (A1)" means that a content of the coloring component other than the component (A1) in the coloring components in the A part is preferably 1% by mass or less, more preferably 0.5% by mass or less, further more preferably 0.2% by mass or less, even more preferably 0% by mass.

In the present specification, the coloring component means a colorant or a precursor thereof. Examples of the coloring component other than the component (A1) include colorants or precursors thereof which are conventionally used in hair dye compositions except for the component (A1) and include oxidation dyes other than the component (A1), direct dyes, and pigments.

The A part substantially contains no oxidizing agent from the viewpoint of suppressing hair damage. The phrase "A part substantially contains no oxidizing agent" means that a content of the oxidizing agent in the A part is preferably less than 0.1% by mass, even more preferably less than 0.01% by mass, further more preferably 0% by mass.

Examples of the oxidizing agent include peroxide such as hydrogen peroxide, urea peroxide, and melamine peroxide. Hydrogen peroxide is preferred. The content of the oxidizing agent means the content of an active ingredient in the oxidizing agent. In using, for example, hydrogen peroxide water, as the oxidizing agent, the content of the oxidizing agent means the content of hydrogen peroxide.

A formulation of the A part is not particularly limited and the A part may be prepared in any formulation, for example, a liquid, a foam, a paste, a cream, a solid, or a powder. The formulation of the A part is preferably a liquid, a foam, a paste, or a cream from the viewpoint of ease of mixing with the B part and from the viewpoint of application to hair by coating, immersion, or the like.

A method for preparing the A part is not particularly limited. This part can be prepared, for example, by blending the component (A1), the component (A2), an aqueous medium, and other optional components and mixing these components with a known stirring apparatus or the like. The A part is preferably prepared in an inert gas (e.g., nitrogen gas) atmosphere from the viewpoint of avoiding polymerization of the component (A1).

[pH]

The pH of the A part is preferably 8.0 or higher, more preferably 9.0 or higher, further more preferably 9.5 or higher, even more preferably 9.8 or higher, from the viewpoint of improving hair dyeability. The pH is preferably 11.0 or lower, more preferably 10.8 or lower, further more preferably 10.5 or lower, from the viewpoint of improving hair dyeability and from the viewpoint of suppressing hair damage.

The pH is a value measured at 25°C and, specifically, can be measured in accordance with a method described in Examples.

(B part)

The B part for use in the step (I) contains the following component (B1) and component (B2):

(B1) an oxidation dye other than the component (A1), and
(B2) an alkali agent.

[Component (B1): oxidation dye other than component (A1)]

The B part contains a component (B1): an oxidation dye other than the component (A1) as a coloring component.
Examples of the oxidation dye for use as the component (B1) include compounds known as precursors or couplers.
Examples of the precursor include one or more members selected from the group consisting of paraphenylenediamine, toluene-2,5-diamine, 2-chloroparaphenylenediamine, N-methoxyethylparaphenylenediamine, N,N-bis(2-hydroxyethyl)paraphenylenediamine, 2-(2-hydroxyethyl)paraphenylenediamine, 2,6-dimethylparaphenylenediamine, 4,4'-diaminodiphenylamine, 1,3-bis(N-(2-hydroxyethyl)-N-(4-aminophenyl)amino)-2-propanol, PEG-3,3,2'-paraphenylenediamine, paraaminophenol, paramethylaminophenol, 3-methyl-4-aminophenol [= 4-aminometacresol], 2-aminomethyl-4-aminophenol, 2-(2-hydroxyethylaminomethyl)-4-aminophenol, orthoaminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-pyrimidinol, 4,5-diamino-1-(4'-chlorobenzyl)pyrazole, 1-hydroxyethyl-4,5-diaminopyrazole, and a salt thereof.
Examples of the coupler include one or more members selected from the group consisting of metaphenylenediamine, 2,4-diaminophenoxyethanol, 2-amino-4-(2-hydroxyethylamino)anisole [= 2-amino-4-(β-hydroxyethyl)aminoanisole], 2,4-diamino-5-methylphenetole, 2,4-diamino-5-(2-hydroxyethoxy)toluene, 2,4-dimethoxy-1,3-diaminobenzene, 2,6-bis(2-hydroxyethylamino) toluene, 2,4-diamino-5-fluorotoluene, 1,3-bis(2,4-diaminophenoxy)propane, metaaminophenol, 2-methyl-5-aminophenol [= 5-aminoorthocresol], 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino)phenol, 2-methyl-4-fluoro-5-aminophenol, resorcinol, 2-methylresorcinol, 4-chlororesorcinol, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl) amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, and a salt thereof.
One or more of these oxidation dyes can be used as the component (B1). Only the precursor may be contained as the component (B1), or both the precursor and the coupler may be contained.
The component (B1) can be appropriately selected in accordance with a desired color and preferably contains one or more members selected from the group consisting of paraaminophenol, toluene-2,5-diamine, 2,4,5,6-tetraaminopyrimidine, 2,5,6-triamino-4-pyrimidinol, 1-hydroxyethyl-4,5-diaminopyrazole, metaaminophenol, and a salt thereof, more preferably contains one or more members selected from the group consisting of paraaminophenol, toluene-2,5-diamine, 2,5,6-triamino-4-pyrimidinol, 1-hydroxyethyl-4,5-diaminopyrazole, and a salt thereof, and further more preferably contains paraaminophenol, from the viewpoint of use in combination with the component (A1).
A content of the component (B1) in the B part is preferably 0.01% by mass or more, more preferably 0.02% by mass or more, further more preferably 0.05% by mass or more, even more preferably 0.10% by mass or more, even more preferably 0.15% by mass or more, from the viewpoint of improvement in hair dyeability and from the viewpoint of easiness in expression of various colors, and preferably 5.0% by mass or less, more preferably 3.0% by mass or less, further more preferably 2.0% by mass or less, from the viewpoint of the solubility and formulation stability of the component (B1).

[Component (B2): alkali agent]

The component (B2) has the same action as that of the component (A2).
Any alkali agent for use in a usual hair dye composition can be used without particular limitations as the component (B2), which may be any of inorganic alkali agents and organic alkali agents. The component (B2) is preferably ammonia or an organic alkali agent from the viewpoint of improvement in hair dyeability.
Examples of the alkali agent which is preferred as the component (B2) include: ammonia; alkanolamine such as mono-, di-, or trimethanolamine, mono-, di-, or triethanolamine, trishydroxymethylaminomethane, and 2-amino-2-methyl-1-propanol; alkylamine such as methylamine, dimethylamine, ethylamine, diethylamine, N-methylethylamine, propylamine, and butylamine; and aralkylamine such as benzylamine, one or more of which can be used. The

alkanolamine, the alkylamine, or the aralkylamine has preferably 1 or more and 10 or less, more preferably 1 or more and 8 or less carbon atoms from the viewpoint of water solubility.

Among those described above, the component (B2) preferably contains one or more members selected from the group consisting of ammonia, alkanolamine, alkylamine, and aralkylamine, more preferably one or more members selected from the group consisting of ammonia and alkanolamine, and further more preferably alkanolamine, from the viewpoint of improvement in hair dyeability and from the viewpoint of easiness in expression of various colors. The component (B2) preferably contains one or more members selected from the group consisting of monoethanolamine, trishydroxymethylaminomethane, and 2-amino-2-methyl-1-propanol, more preferably contains one or more members selected from the group consisting of monoethanolamine and 2-amino-2-methyl-1-propanol, and further more preferably contains monoethanolamine, as the alkanolamine from the viewpoint of improvement in hair dyeability, from the viewpoint of easiness in expression of various colors.

A content of the alkanolamine in the component (B2) is preferably 70% by mass or more, more preferably 80% by mass or more, further more preferably 90% by mass or more, and 100% by mass or less, from the viewpoint of improvement in hair dyeability and from the viewpoint of easiness in expression of various colors.

The component (A2) and the component (B2) may be the same type of alkali agent or may be different types of alkali agents and are preferably the same type of alkali agent from the viewpoint of the miscibility between the A part and the B part and from the viewpoint of ease of pH adjustment.

Specifically, each of the component (A2) and the component (B2) preferably contains one or more members selected from the group consisting of ammonia, alkanolamine, alkylamine, and aralkylamine, more preferably one or more members selected from the group consisting of ammonia and alkanolamine, and further more preferably alkanolamine.

A content of the component (B2) in the B part is preferably 0.01% by mass or more, more preferably 0.1% by mass or more, further more preferably 0.5% by mass or more, even more preferably 1.0% by mass or more, even more preferably 2.0% by mass or more, from the viewpoint of improvement in hair dyeability, and preferably 10% by mass or less, more preferably 7.5% by mass or less, further more preferably 5.0% by mass or less, from the viewpoint of suppressing hair damage.

[Component (B3): antioxidant]

The B part preferably further contains an antioxidant as a component (B3) from the viewpoint of suppressing the oxidative polymerization of the component (B1) outside hair.

Examples of the antioxidant include sulfurous acid, pyrosulfurous acid, hydrogen sulfite, sulfur dioxide, L-ascorbic acid, thioglycolic acid, L-cysteine, N-acetyl-L-cysteine, and a salt thereof, and a derivative thereof. Examples of the salt include alkali metals such as sodium and potassium.

Among those described above, the antioxidant is preferably one or more members selected from the group consisting of sulfite, pyrosulfite, hydrosulfite, sulfur dioxide, L-ascorbic acid, and L-ascorbate, more preferably one or more members selected from the group consisting of sodium sulfite and sodium L-ascorbate, further more preferably sodium sulfite, from the viewpoint of the effect of suppressing the oxidative polymerization of the component (B1) outside hair.

When the B part contains the antioxidant(B3), a content of the component (B3) in the B part is preferably 0.01% by mass or more, more preferably 0.05% by mass or more, further more preferably 0.1% by mass or more, even more preferably 0.2% by mass or more, and preferably 10% by mass or less, more preferably 5.0% by mass or less, further more preferably 3.0% by mass or less, even more preferably 2.0% by mass or less, even more preferably 1.0% by mass or less, even more preferably 0.5% by mass or less, from the viewpoint of improvement in the effect of suppressing the oxidative polymerization of the component (B1) outside hair.

When the B part contains the antioxidant(B3), a mass ratio of the component (B3) to the component (B1) [(B3)/(B1)] in the B part is preferably 0.05 or more, more preferably 0.1 or more, further more preferably 0.2 or more, from the viewpoint of the effect of suppressing the oxidative polymerization of the component (B1) outside hair. The mass ratio is preferably 10 or less, more preferably 5.0 or less, further more preferably 3.0 or less, even more preferably 2.5 or less, from the viewpoint of improvement in hair dyeability.

[pH adjuster]

The B part preferably further contains a pH adjuster from the viewpoint of adjusting pH to a desired range described hereinafter, accelerating the polymerization reaction within hair of the component (B1), and improving hair dyeability.

Since the B part contains the alkali agent as the component (B2), the pH adjuster for use in the B part is preferably a protonating agent. The protonating agent may be any of monobasic acids and polybasic acids or may be any of organic acids (having 1 or more and 8 or less carbon atoms, except for ascorbic acid) and inorganic acids.

Examples of the protonating agent include one or more members selected from the group consisting of

hydrochloric acid, sulfuric acid, phosphoric acid, formic acid, acetic acid, and citric acid. One or more members selected from the group consisting of phosphoric acid and citric acid is preferred from the viewpoint of adjusting pH of the B part to a range described hereinafter.

The pH adjusters for use in the A part and the B part may be the same type of pH adjuster or may be different types of pH adjusters and are preferably the same type of pH adjuster from the viewpoint of the miscibility between the A part and the B part and from the viewpoint of ease of pH adjustment.

When the B part contains the pH adjuster, a content of the pH adjuster in the B part is preferably 0.05% by mass or more, more preferably 0.1% by mass or more, further more preferably 0.2% by mass or more, and preferably 5.0% by mass or less, more preferably 4.0% by mass or less, further more preferably 3.5% by mass or less, even more preferably 2.5% by mass or less, even more preferably 1.5% by mass or less, from the viewpoint of adjusting pH of the B part to a range described hereinafter.

[Aqueous medium]

The B part usually further contains an aqueous medium. Examples of the aqueous medium include: water; lower alcohols such as ethanol and isopropyl alcohol; and low-molecular diol and triol having 6 or less carbon atoms, such as 1,3-butylene glycol, glycerin, ethylene glycol, and propylene glycol. The aqueous medium is preferably one or more members selected from the group consisting of water and ethanol, more preferably water, from the viewpoint of the solubility of the component (B1) and the component (B2), from the viewpoint of affinity for hair, and from the viewpoint of suppressing hair damage.

A content of the aqueous medium in the B part is usually in the range of from 1 to 99% by mass and can be appropriately selected in accordance with a formulation of the B part, and the like. The content of the aqueous medium in the B part is preferably 50% by mass or more, more preferably 70% by mass or more, further more preferably 80% by mass or more, from the viewpoint of ease of mixing with the A part, and preferably 98% by mass or less from the viewpoint of easiness in representation of various colors.

The content of the aqueous medium in the B part may be a balance for the component (B1), the component (B2), the antioxidant, and the pH adjuster.

[Other components]

The B part may appropriately contain, in addition to the components described above, components which are conventionally used in hair dye compositions, without impairing the object of the present invention. Examples of the components include surfactants, viscosity adjusters, silicone, aromatic alcohols, coloring components other than the component (B1), polymers, oil agents, anti-dandruff agents, vitamin preparations, germicides, anti-inflammatory agents, antiseptics, chelating agents, humectants, pearlescent agents, a ceramide, fragrances, and ultraviolet absorbers.

The B part may contain a coloring component other than the component (B1). However, according to the hair dyeing method of the present invention, the B part may substantially contain no coloring component other than the component (B1). The phrase "substantially contain no coloring component other than the component (B1)" means that a content of the coloring component other than the component (B1) in the coloring components in the B part is preferably 1% by mass or less, more preferably 0.5% by mass or less, further more preferably 0.2% by mass or less, even more preferably 0% by mass.

Examples of the coloring component other than the component (B1) include colorants or precursors thereof which are conventionally used in hair dye compositions except for the component (B1) and include the component (A1), direct dyes, and pigments.

The B part substantially contains no oxidizing agent from the viewpoint of suppressing hair damage. The phrase "B part substantially contains no oxidizing agent" means that a content of the oxidizing agent in the B part is preferably less than 0.1% by mass, even more preferably less than 0.01% by mass, further more preferably 0% by mass.

Examples of the oxidizing agent include peroxide such as hydrogen peroxide, urea peroxide, and melamine peroxide. Hydrogen peroxide is preferred. The content of the oxidizing agent means the content of an active ingredient in the oxidizing agent. In using, for example, hydrogen peroxide water, as the oxidizing agent, the content of the oxidizing agent means the content of hydrogen peroxide.

A formulation of the B part is not particularly limited and may be any formulation, for example, a liquid, a foam, a paste, a cream, a solid, or a powder. The formulation of the B part is preferably a liquid, a paste, or a cream, more preferably a liquid, from the viewpoint of ease of mixing with the A part and from the viewpoint of application to hair by coating, immersion, or the like.

The A part and the B part may have the same formulation or different formulations and preferably have the same formulation from the viewpoint of ease of mixing.

A method for preparing the B part is not particularly limited. This part can be prepared, for example, by blending

the component (B1), the component (B2), an aqueous medium, and other optional components and mixing these components with a known stirring apparatus or the like.

[pH]

pH of the B part is preferably 8.0 or higher, more preferably 9.0 or higher, further more preferably 9.5 or higher, even more preferably 9.8 or higher, from the viewpoint of improving hair dyeability. The pH is preferably 11.0 or lower, more preferably 10.8 or lower, further more preferably 10.5 or lower, from the viewpoint of improving hair dyeability and from the viewpoint of suppressing hair damage.
The pH is a value measured at 25°C and, specifically, can be measured in accordance with a method described in Examples.
The step (I) involves mixing the A part with the B part before use to prepare a hair dye composition. In the present specification, the phrase "mixing the A part with the B part before use" means that the A part is mixed with the B part before the hair dye composition is applied to hair, and means in the present invention that the mixing is performed immediately before the step (II).
Mixing of the A part with the B part can be performed by a routine method at room temperature in atmosphere. In this mixing, for example, the A part and the B part can be prepared in separate containers, taken out of their respective containers where the parts have been prepared, and mixed in another container.
A mix ratio of the A part and the B part in the step (I) is such that a molar ratio of the component (A1) to the component (B1), [(A1)/(B1)], is from 10/90 to 70/30, preferably from 20/80 to 70/30, more preferably from 30/70 to 70/30, further more preferably from 30/70 to 60/40, even more preferably from 40/60 to 60/40, from the viewpoint of improvement in hair dyeability and from the viewpoint of easiness in expression of various colors. When the molar ratio [(A1)/(B1)] is 10/90 or more, hair dyeability is improved. When the molar ratio is 70/30 or less, expression of various colors is easy.
A formulation of the hair dye composition obtained by mixing the A part with the B part in the step (I) is appropriately determined in accordance with the formulations of the A part and the B part. The formulation of the hair dye composition is preferably a liquid, a paste, or a cream, more preferably a liquid.
The pH at 25°C of the hair dye composition obtained by mixing the A part with the B part in the step (I) is preferably 8.0 or higher, more preferably 9.0 or higher, further more preferably 9.5 or higher, even more preferably 9.8 or higher, from the viewpoint of improving hair dyeability. The pH is preferably 11.0 or lower, more preferably 10.8 or lower, further more preferably 10.5 or lower, from the viewpoint of improving hair dyeability and from the viewpoint of suppressing hair damage.
The pH is a value measured at 25°C and, specifically, can be measured in accordance with a method described in Examples.

<Step (II) >

The step (II) involves applying the hair dye composition prepared in the step (I) to hair.
Examples of a method for applying the hair dye composition to hair may not be limited as long as the method facilitates contact of the hair dye composition with the hair. Examples thereof include a method of coating hair in a dry state or a wet state with the composition, and a method of immersing hair in a dry state or a wet state in the composition. Among those described above, a method of coating hair in a dry state with the hair dye composition is preferred.
An amount of the hair dye composition applied to the hair is a bath ratio (dry mass of the hair : mass of the hair dye composition) of preferably from 1 : 0.2 to 1 : 20, more preferably from 1 : 0.5 to 1 : 10, further more preferably from 1 : 0.5 to 1 : 5, even more preferably from 1 : 0.8 to 1 : 3, from the viewpoint of balance between hair dyeability and economic performance.
The hair to which the hair dye composition is to be applied is preferably hair of the head and can be at least a portion of the hair of the head.
In coating the hair with the hair dye composition in the step (II), coating time is preferably 10 seconds or longer, more preferably 20 seconds or longer, from the viewpoint of improvement in hair dyeability, and preferably 10 minutes or shorter, more preferably 5 minutes or shorter, from the viewpoint of reducing burden on a practitioner.
After the step (II) of applying the hair dye composition to hair, a step of leaving the resultant to stand is preferably further performed. In this respect, time for which the resultant is left to stand is preferably 1 minute or longer, more preferably 3 minutes or longer, from the viewpoint of improvement in hair dyeability. The time is preferably 30 minutes or shorter, more preferably 20 minutes or shorter, from the viewpoint of reducing burden on a subject.
Temperature in performing the step (II) is not particularly limited and is preferably 40°C or lower, more preferably 35°C or lower, from the viewpoint of the handleability of the hair dye composition. The temperature is preferably 25°C or higher, more preferably 30°C or higher, from the viewpoint of improvement in hair dyeability.

<Rinsing step>

The method of the present invention preferably includes, after the step (II), a step of rinsing the hair dye composition applied to the hair (hereinafter, also simply referred to as a "rinsing step"). The rinsing step is performed, for example, by washing off the hair dye composition applied to the hair with water. Temperature of the water is not particularly limited, and warm water of 35 to 45°C is preferred. Time for which the composition is washed off with warm water during the rinsing is not particularly limited and is preferably 15 to 45 seconds.

<Drying step>

The method of the present invention preferably includes, after the rinsing step, a step of drying the hair (hereinafter, also simply referred to as a "drying step"). The step of drying the hair is a step of reducing an amount of water in the hair. The step of drying the hair includes, for example, performing one or more drying treatments selected from the group consisting of towel drying, blow (cold air or hot air) drying, and air drying in order to actively reduce the amount of water in the hair. Two or more of these drying treatments are preferably performed in combination. Among them, a combination of towel drying and cold-air blow drying is more preferred, and towel drying followed by cold-air blow drying is further more preferred.

The method of the present invention has no step of applying an oxidizing agent to hair from the viewpoint of suppressing hair damage.

In the present specification, the phrase "comprise no step of applying an oxidizing agent to hair" refers to absence of the step of applying a solution or a composition containing 0.01% by mass or more of the oxidizing agent to hair.

The "composition containing 0.01% by mass or more of the oxidizing agent" also includes a hair dye composition. Thus, the hair dye composition to be applied to hair and the A part and the B part constituting the hair dye composition substantially contain no oxidizing agent.

The phrase "applying an oxidizing agent to hair" refers to, for example, contacting a solution or a composition containing 0.01% by mass or more of the oxidizing agent with hair before, during, and after dyeing by coating, immersion, or the like. In this context, examples of the oxidizing agent include peroxide such as hydrogen peroxide, urea peroxide, and melamine peroxide. Hydrogen peroxide is preferred.

Hair damage of the hair thus treated in accordance with the method of the present invention is suppressed. The hair damage can be evaluated from finger combability of the hair treated by hair dyeing (feel evaluation). Specifically, the hair damage can be evaluated in accordance with a method described in Examples.

The hair damage may be evaluated from an amount of cysteic acid generated (see, for example, Reference 1 (Teruo Horiuchi, Fragrance Journal, December 2019, vol. 18, No. 12, p. 69-86)), in addition to the feel evaluation.

The evaluation from the amount of cysteic acid generated can be performed, for example, as follows; a 10 cm long tress is subjected to each hair dyeing treatment, and five strands of hair are collected. An ATR-IR spectrum in a central part of the collected hair is measured under conditions involving a wavenumber resolution of 4 $cm^{-1}$ and an integrated number of scans of 4 times by the universal ATR method using Spectrum 3 from PerkinElmer, Inc. In this respect, the S=O stretching vibration of a sulfonic acid group ($-SO_3H$) of cysteic acid generated in association with the hair dyeing treatment appears around 1040 $cm^{-1}$. The whole spectrum is standardized such that the peak height of amide (around 1 650 $cm^{-1}$) is 1. Then, data points at 1033 $cm^{-1}$ and 1049 $cm^{-1}$ are connected through a straight line, and an area value of a portion above the line is calculated. A value obtained by multiplying the area value by 100 is regarded as cysteic acid signal intensity, which is used as the amount of cysteic acid generated. The hair dyeing treatment of the present invention can perform hair dyeing treatment while sufficiently reducing the amount of cysteic acid generated.

The method of the present invention can easily express various colors, as described above.

The variety of this color expression can be evaluated using a difference in chroma C value ($\Delta C$) between a hair dyeing treatment with each of the A part and the B part alone and a hair dyeing treatment with the hair dye composition obtained by mixing the A part with the B part. That is, the variety can be evaluated by calculating a difference $\Delta C_A$ between a chroma C value of hair treated by hair dyeing with only the A part and a chroma C value of hair treated by hair dyeing with the hair dye composition, and a difference $\Delta C_B$ between a chroma C value of hair treated by hair dyeing with only the B part and the chroma C value of hair treated by hair dyeing with the hair dye composition. Specifically, the variety can be evaluated in accordance with a method described in Examples. In this respect, numeric values of these differences preferably satisfy respective predetermined ranges. The numeric values are, for example, preferably $\Delta C_A$ of from 1.0 to 12.0 and $\Delta C_B$ of from 1.7 to 12.0, more preferably $\Delta C_A$ of from 2.5 to 11.0 and $\Delta C_B$ of from 2.0 to 10.0, further more preferably $\Delta C_A$ of from 5.0 to 9.0 and $\Delta C_B$ of from 4.0 to 7.5.

[Kit for dyeing hair]

The present invention also provides a kit for dyeing hair, containing:

an A part comprising the following component (A1) and component (A2):

(A1) a compound of the following formula (1) or a salt thereof:

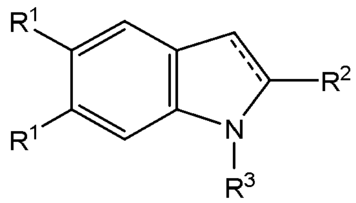

(1)

wherein the broken line represents presence or absence of a π bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and
(A2) an alkali agent; and

a B part containing the following component (B1) and component (B2):

(B1) an oxidation dye other than the component (A1), and
(B2) an alkali agent,

the kit containing no preparation containing an oxidizing agent.

The A part, the B part, and their preferred forms are the same as those described above, and neither the A part nor the B part contains an oxidizing agent. The oxidizing agent and specific examples thereof are the same as those described above.

The kit for dyeing hair of the present invention may not be limited as long as the kit contains the A part and the B part. Examples thereof include two-part kits in which the A part and the B part are individually enclosed in their respective containers. The shapes of the containers are not particularly limited and can be appropriately selected in accordance with formulations. Examples thereof include bottles, tubes, dispensers, and aerosols.

The kit for dyeing hair of the present invention may further have a preparation other than the A part and the B part. Preferably, the preparation substantially contains no oxidizing agent.

The kit for dyeing hair may further have, if necessary, a comb, a brush, or the like for coating hair with the hair dye composition obtained by mixing the A part with the B part, for example.

The kit for dyeing hair of the present invention can be used, preferably, in accordance with the method described in the method for dyeing hair mentioned above.

Hereinafter, in relation to the embodiments mentioned above, the present invention further discloses the following.

<1> A method for dyeing hair, comprising the following step (I) and (II) in order:

(I) mixing an A part with a B part before use to prepare a hair dye composition,

the A part comprising the following component (A1) and component (A2):

(A1) a compound of the following formula (1) or a salt thereof:

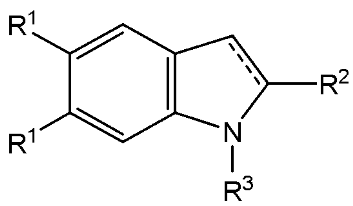

(1)

wherein the broken line represents presence or absence of a π bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an

an ethyl group, and
(A2) an alkali agent,

the B part comprising the following component (B1) and component (B2):

(B1) an oxidation dye other than the component (A1), and
(B2) an alkali agent,; and

(II) applying the hair dye composition to hair, wherein

a mix ratio of the A part and the B part in the step (I) is such that a molar ratio of the component (A1) to the component (B1), [(A1)/(B1)], is from 10/90 to 70/30, and
the method for dyeing hair comprises no step of applying an oxidizing agent to hair.

<2> The method for dyeing hair according to <1>, wherein the component (A1) is one or more members selected from the group consisting of 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-hydroxyindole, 5-acetoxy-6-hydroxyindole-2-carboxylic acid, 5,6-dihydroxyindoline, 5,6-dihydroxyindoline-2-carboxylic acid, methyl 5,6-dihydroxyindoline-2-carboxylate, ethyl 5,6-dihydroxyindoline-2-carboxylate, N-methyl-5,6-dihydroxyindoline, N-methyl-5,6-dihydroxyindoline-2-carboxylic acid, N-ethyl-5,6-dihydroxyindoline, N-ethyl-5,6-dihydroxyindoline-2-carboxylic acid, N-acetyl-5,6-dihydroxyindoline, N-acetyl-5,6-dihydroxyindoline-2-carboxylic acid, 5-acetoxy-6-hydroxyindoline, and 5-acetoxy-6-hydroxyindoline-2-carboxylic acid, and hydrochloride, hydrobromide, sulfate, phosphate, acetate, propionate, lactate, and citrate of each of these compounds, preferably hydrobromide of each of these compounds.
<3> The method for dyeing hair according to <1> or <2>, wherein the component (A1) is preferably one or more members selected from the group consisting of 5,6-dihydroxyindole, 5,6-dihydroxyindole-2-carboxylic acid, 5,6-dihydroxyindoline, 5,6-dihydroxyindoline-2-carboxylic acid, and a salt thereof, more preferably one or more members selected from the group consisting of 5,6-dihydroxyindole, 5,6-dihydroxyindole-2-carboxylic acid, and 5,6-dihydroxyindoline hydrobromide, further more preferably one or more members selected from the group consisting of 5,6-dihydroxyindole and 5,6-dihydroxyindole-2-carboxylic acid, even more preferably comprises 5,6-dihydroxyindole, and even more preferably comprises 5,6-dihydroxyindole and 5,6-dihydroxyindole-2-carboxylic acid.
<4> The method for dyeing hair according to any one of <1> to <3>, wherein when $R^2$ in the formula (1) of the component (A1) is -COOH, the salt of the compound of the formula (1) is carboxylate thereof (wherein $R^2$ is $-COO^-X^+$ ($X^+$ is a cation)).
<5> The method for dyeing hair according to any one of <1> to <4>, wherein when the component (A1) comprises 5,6-dihydroxyindole and 5,6-dihydroxyindole-2-carboxylic acid, a molar ratio of the 5,6-dihydroxyindole and the 5,6-dihydroxyindole-2-carboxylic acid is preferably in the range of from 50 : 50 to 99 : 1, more preferably in the range of from 80 : 20 to 99 : 1, further more preferably in the range of from 85 : 15 to 95 : 5.
<6> The method for dyeing hair according to any one of <1> to <5>, wherein a content of the component (A1) is preferably 0.02% by mass or more, more preferably 0.05% by mass or more, further more preferably 0.1% by mass or more, and preferably 5.0% by mass or less, more preferably 3.0% by mass or less, further more preferably 2.0% by mass or less, even more preferably 1% by mass or less, even more preferably 0.8% by mass or less, even more preferably 0.5% by mass or less.
<7> The method for dyeing hair according to any one of <1> to <6>, wherein the component (B1) comprises at least one or more members selected from the group consisting of a precursor and a coupler.
<8> The method for dyeing hair according to <7>, wherein the precursor comprises one or more members selected from the group consisting of paraphenylenediamine, toluene-2,5-diamine, 2-chloro-paraphenylenediamine, N-methoxyethylparaphenylenediamine, N,N-bis(2-hydroxyethyl)paraphenylenediamine, 2-(2-hydroxyethyl)paraphenylenediamine, 2,6-dimethylparaphenylenediamine, 4,4'-diaminodiphenylamine, 1,3-bis(N-(2-hydroxyethyl)-N-(4-aminophenyl)amino)-2-propanol, PEG-3,3,2'-paraphenylenediamine, paraaminophenol, paramethylaminophenol, 3-methyl-4-aminophenol [= 4-aminometacresol], 2-aminomethyl-4-aminophenol, 2-(2-hydroxyethylaminomethyl)-4-aminophenol, orthoaminophenol, 2-amino-5-methylphenol, 2-amino-6-methylphenol, 2-amino-5-acetamidophenol, 3,4-diaminobenzoic acid, 5-aminosalicylic acid, 2,4,5,6-tetraaminopyrimidine, 2,5,6-triamino-4-pyrimidinol, 4,5-diamino-1-(4'-chlorobenzyl)pyrazole, 1-hydroxyethyl-4,5-diaminopyrazole, and a salt thereof.
<9> The method for dyeing hair according to <7> or <8>, wherein the coupler comprises one or more members selected from the group consisting of metaphenylenediamine, 2,4-diaminophenoxyethanol, 2-amino-4-(2-hydroxyethylamino)anisole [= 2-amino-4-(β-hydroxyethyl)aminoanisole], 2,4-diamino-5-methylphenetole, 2,4-diamino-5-(2-hydroxyethoxy)toluene, 2,4-dimethoxy-1,3-diaminobenzene, 2,6-bis(2-hydroxyethylamino) toluene, 2,4-diamino-5-fluorotoluene, 1,3-bis(2,4-diaminophenoxy)propane, metaaminophenol, 2-methyl-5-aminophenol [= 5-aminoorthocresol], 2-methyl-5-(2-hydroxyethylamino)phenol, 2,4-dichloro-3-aminophenol, 2-chloro-3-amino-6-methylphenol, 2-methyl-4-chloro-5-aminophenol, N-cyclopentyl-metaaminophenol, 2-methyl-4-methoxy-5-(2-hydroxyethylamino)phenol, 2-methyl-4-fluoro-5-aminophenol, resorcinol, 2-methylresorcinol, 4-chlororesorcinol, 1-naphthol, 1,5-dihydroxynaphthalene, 1,7-dihydroxynaphthalene, 2,7-dihydroxynaphthalene, 2-isopropyl-5-methylphenol, 4-hydroxyindole, 5-hydroxyindole, 6-hydroxyindole, 7-hydroxyindole, 6-hydroxybenzomorpholine, 3,4-methylenedioxyphenol, 2-bromo-4,5-methylenedioxyphenol, 3,4-methylenedioxyaniline, 1-(2-hydroxyethyl)amino-3,4-methylenedioxybenzene, 2,6-dihydroxy-3,4-dimethylpyridine, 2,6-dimethoxy-3,5-diaminopyridine, 2,3-diamino-6-methoxypyridine, 2-methylamino-3-amino-6-methoxypyridine, 2-amino-3-hydroxypyridine, 2,6-diaminopyridine, and a salt thereof.

<10> The method for dyeing hair according to any one of <1> to <9>, wherein the component (B1) preferably comprises one or more members selected from the group consisting of paraaminophenol, toluene-2,5-diamine, 2,4,5,6-tetraaminopyrimidine, 2,5,6-triamino-4-pyrimidinol, 1-hydroxyethyl-4,5-diaminopyrazole, metaaminophenol, and a salt thereof, and more preferably comprises paraaminophenol.

<11> The method for dyeing hair according to any one of <1> to <10>, wherein a content of the component (B1) in the B part is preferably 0.01% by mass or more, more preferably 0.02% by mass or more, further more preferably 0.05% by mass or more, even more preferably 0.10% by mass or more, even more preferably 0.15% by mass or more, and preferably 5.0% by mass or less, more preferably 3.0% by mass or less, further more preferably 2.0% by mass or less.

<12> The method for dyeing hair according to any one of <1> to <11>, wherein the component (A2) and/or the component (B2) is preferably ammonia or an organic alkali agent, more preferably comprises at least one or more members selected from the group consisting of ammonia, alkanolamine, alkylamine, and aralkylamine, further more preferably comprises one or more members selected from the group consisting of ammonia and alkanolamine, and even more preferably comprises alkanolamine.

<13> The method for dyeing hair according to <12>, wherein the alkanolamine, the alkylamine, or the aralkylamine has preferably 1 or more and 10 or less, more preferably 1 or more and 8 or less carbon atoms.

<14> The method for dyeing hair according to <12> or <13>, wherein the component (A2) and/or the component (B2) preferably comprises one or more members selected from the group consisting of monoethanolamine, trishydroxymethylaminomethane, and 2-amino-2-methyl-1-propanol, more preferably comprises one or more members selected from the group consisting of monoethanolamine and 2-amino-2-methyl-1-propanol, and further more preferably comprises monoethanolamine.

<15> The method for dyeing hair according to any one of <12> to <14>, wherein a content of the alkanolamine in the component (A2) and/or the component (B2) is 70% by mass or more, more preferably 80% by mass or more, further more preferably 90% by mass or more, and 100% by mass or less.

<16> The method for dyeing hair according to any one of <1> to <15>, wherein a content of the component (A2) in the A part and/or the component (B2) in the B part is preferably 0.01% by mass or more, more preferably 0.1% by mass or more, further more preferably 0.5% by mass or more, even more preferably 1.0% by mass or more, even more preferably 2.0% by mass or more, and preferably 10% by mass or less, more preferably 7.5% by mass or less, further more preferably 5.0% by mass or less.

<17> The method for dyeing hair according to any one of <1> to <16>, wherein the component (A2) and the component (B2) are the same type of alkali agent.

<18> The method for dyeing hair according to any one of <1> to <17>, wherein the A part further comprises an antioxidant as a component (A3).

<19> The method for dyeing hair according to <18>, wherein the component (A3) comprises at least one or more members selected from the group consisting of sulfurous acid, pyrosulfurous acid, hydrogen sulfite, sulfur dioxide, L-ascorbic acid, thioglycolic acid, L-cysteine, N-acetyl-L-cysteine, and a salt thereof, and a derivative thereof, preferably comprises at least one member selected from the group consisting of sulfite, pyrosulfite, hydrosulfite, sulfur dioxide, L-ascorbic acid, and L-ascorbate, is more preferably one or more members selected from the group consisting of sodium sulfite and sodium L-ascorbate, and is further more preferably sodium sulfite.

<20> The method for dyeing hair according to <18> or <19>, wherein a content of the component (A3) in the A part is preferably 0.01% by mass or more, more preferably 0.05% by mass or more, further more preferably 0.1% by mass or more, even more preferably 0.2% by mass or more, and preferably 10% by mass or less, more preferably 5.0% by mass or less, further more preferably 3.0% by mass or less, even more preferably 2.0% by mass or less, even more preferably 1.0% by mass or less, even more preferably 0.5% by mass or less.

<21> The method for dyeing hair according to any one of <18> to <20>, wherein the component (A3) is contained, and a mass ratio of the component (A3) to the component (A1) [(A3)/(A1)] in the A part is preferably 0.05 or more, more preferably 0.1 or more, further more preferably 0.2 or more, even more preferably 0.3 or more, even more preferably 0.5 or more, even more preferably 0.8 or more, even more preferably 1.0 or more, even more preferably 1.2 or more,

and preferably 10 or less, more preferably 5.0 or less, further more preferably 3.0 or less, even more preferably 2.0 or less.

<22> The method for dyeing hair according to any one of <1> to <21>, wherein the B part further comprises an antioxidant as a component (B3).

<23> The method for dyeing hair according to <22>, wherein the component (B3) comprises at least one or more members selected from the group consisting of sulfurous acid, pyrosulfurous acid, hydrogen sulfite, sulfur dioxide, L-ascorbic acid, thioglycolic acid, L-cysteine, N-acetyl-L-cysteine, and a salt thereof, and a derivative thereof, preferably comprises at least one member selected from the group consisting of sulfite, pyrosulfite, hydrosulfite, sulfur dioxide, L-ascorbic acid, and L-ascorbate, is more preferably one or more members selected from the group consisting of sodium sulfite and sodium L-ascorbate, and is further more preferably sodium sulfite.

<24> The method for dyeing hair according to <22> or <23>, wherein a content of the component (B3) in the B part is preferably 0.01% by mass or more, more preferably 0.05% by mass or more, further more preferably 0.1% by mass or more, even more preferably 0.2% by mass or more, and preferably 10% by mass or less, more preferably 5.0% by mass or less, further more preferably 3.0% by mass or less, even more preferably 2.0% by mass or less, even more preferably 1.0% by mass or less, even more preferably 0.5% by mass or less.

<25> The method for dyeing hair according to any one of <22> to <24>, wherein the component (B3) is contained, and a mass ratio of the component (B3) to the component (B1) [(B3)/(B1)] in the B part is preferably 0.05 or more, more preferably 0.1 or more, further more preferably 0.2 or more, and preferably 10 or less, more preferably 5.0 or less, further more preferably 3.0 or less, even more preferably 2.5 or less.

<26> The method for dyeing hair according to any one of <1> to <25>, wherein the A part and/or the B part further comprises a pH adjuster, and the pH adjuster is preferably one or more protonating agents selected from the group consisting of a monobasic acid and a polybasic acid, or one or more protonating agents selected from the group consisting of an organic acid (having 1 or more and 8 or less carbon atoms, except for ascorbic acid) and an inorganic acid, more preferably one or more members selected from the group consisting of hydrochloric acid, sulfuric acid, phosphoric acid, formic acid, acetic acid, and citric acid as the protonating agent, further more preferably one or more members selected from the group consisting of phosphoric acid and citric acid.

<27> The method for dyeing hair according to <26>, wherein a content of the pH adjuster is preferably 0.05% by mass or more, more preferably 0.1% by mass or more, further more preferably 0.2% by mass or more, and preferably 5.0% by mass or less, more preferably 4.0% by mass or less, further more preferably 3.5% by mass or less, even more preferably 2.5% by mass or less, even more preferably 1.5% by mass or less.

<28> The method for dyeing hair according to <26> or <27>, wherein the pH adjusters for use in the A part and the B part are the same type of pH adjuster.

<29> The method for dyeing hair according to any one of <1> to <28>, wherein pH of the A part and/or the B part is preferably 8.0 or higher, more preferably 9.0 or higher, further more preferably 9.5 or higher, even more preferably 9.8 or higher, and preferably 11.0 or lower, more preferably 10.8 or lower, further more preferably 10.5 or lower.

<30> The method for dyeing hair according to any one of <1> to <29>, wherein the A part and/or the B part further comprises an aqueous medium, and the aqueous medium is preferably one or more members selected from the group consisting of water, a lower alcohol, and low-molecular diol and triol having 6 or less carbon atoms, more preferably one or more members selected from the group consisting of water and ethanol, further more preferably water.

<31> The method for dyeing hair according to <30>, wherein a content of the aqueous medium in the A part and/or the B part is in the range of from 1 to 99% by mass, preferably 50% by mass or more, more preferably 70% by mass or more, further more preferably 80% by mass or more, and preferably 98% by mass or less.

<32> The method for dyeing hair according to any one of <1> to <31>, wherein the A part further comprises at least one member selected from the group consisting of a surfactant, a viscosity adjuster, silicone, an aromatic alcohol, a coloring component other than the component (A1), a polymer, an oil agent, an anti-dandruff agent, a vitamin preparation, a germicide, an anti-inflammatory agent, an antiseptic, a chelating agent, a humectant, a pearlescent agent, a ceramide, a fragrance, and an ultraviolet absorber.

<33> The method for dyeing hair according to any one of <1> to <32>, wherein the A part substantially contains no coloring component other than the component (A1), and a content of the coloring component other than the component (A1) in the coloring components in the A part is preferably 1% by mass or less, more preferably 0.5% by mass or less, further more preferably 0.2% by mass or less, even more preferably 0% by mass.

<34> The method for dyeing hair according to any one of <1> to <33>, wherein the B part further comprises at least one member selected from the group consisting of a surfactant, a viscosity adjuster, silicone, an aromatic alcohol, a coloring component other than the component (B1), a polymer, an oil agent, an anti-dandruff agent, a vitamin preparation, a germicide, an anti-inflammatory agent, an antiseptic, a chelating agent, a humectant, a pearlescent agent, a ceramide, a fragrance, and an ultraviolet absorber.

<35> The method for dyeing hair according to any one of <1> to <34>, wherein the B part substantially contains no coloring component other than the component (B1), and a content of the coloring component other than the

component (B1) in the coloring components in the B part is preferably 1% by mass or less, more preferably 0.5% by mass or less, further more preferably 0.2% by mass or less, even more preferably 0% by mass.

<36> The method for dyeing hair according to any one of <1> to <35>, wherein the A part and/or the B part substantially contains no oxidizing agent, and a content of the oxidizing agent in the A part and/or the B part is preferably less than 0.1% by mass, even more preferably less than 0.01% by mass, further more preferably 0% by mass.

<37> The method for dyeing hair according to <36>, wherein the oxidizing agent is peroxide, preferably hydrogen peroxide.

<38> The method for dyeing hair according to any one of <1> to <37>, wherein a formulation of the A part and/or the B part is a liquid, a foam, a paste, a cream, a solid, or a powder, preferably a liquid, a paste, or a cream, more preferably a liquid.

<39> The method for dyeing hair according to <38>, wherein the formulations of the A part and the B part are the same formulation.

<40> The method for dyeing hair according to any one of <1> to <39>, wherein the A part is prepared by blending and mixing the component (A1), the component (A2), an aqueous medium, and other optional components, and preferably prepared in an inert gas atmosphere.

<41> The method for dyeing hair according to any one of <1> to <40>, wherein the B part is prepared by blending and mixing the component (B1), the component (B2), an aqueous medium, and other optional components.

<42> The method for dyeing hair according to any one of <1> to <41>, wherein in the step (I), the A part is mixed with the B part immediately before the step (II) of applying the hair dye composition to hair.

<43> The method for dyeing hair according to any one of <1> to <42>, wherein the mixing before use in the step (I) is performed by taking the A part and the B part out of their respective containers where the parts have been prepared, and mixing the parts in an another container.

<44> The method for dyeing hair according to any one of <1> to <43>, wherein a formulation of the hair dye composition obtained in the step (I) is preferably a liquid, a paste, or a cream, more preferably a liquid.

<45> The method for dyeing hair according to any one of <1> to <44>, wherein pH at 25°C of the hair dye composition is preferably 8.0 or higher, more preferably 9.0 or higher, further more preferably 9.5 or higher, even more preferably 9.8 or higher, and preferably 11.0 or lower, more preferably 10.8 or lower, further more preferably 10.5 or lower.

<46> The method for dyeing hair according to any one of <1> to <45>, wherein in the step (II), a method for applying the hair dye composition to hair is a method of contacting the hair dye composition with the hair, preferably a method of coating hair in a dry state or a wet state with the hair dye composition or a method of immersing hair in a dry state or a wet state in the hair dye composition.

<47> The method for dyeing hair according to any one of <1> to <46>, wherein in the step (II), an amount of the hair dye composition applied to the hair is a bath ratio (dry mass of the hair : mass of the hair dye composition) of preferably from 1 : 0.2 to 1 : 20, more preferably from 1 : 0.5 to 1 : 10, further more preferably from 1 : 0.5 to 1 : 5, even more preferably from 1 : 0.8 to 1 : 3.

<48> The method for dyeing hair according to any one of <1> to <47>, wherein in coating hair with the hair dye composition in the step (II), coating time is preferably 10 seconds or longer, more preferably 20 seconds or longer, and preferably 10 minutes or shorter, more preferably 5 minutes or shorter.

<49> The method for dyeing hair according to any one of <1> to <48>, further comprising, after the step (II) of applying the hair dye composition to hair, the step of leaving the resultant, wherein time for which the resultant is left to stand is preferably 1 minute or longer, more preferably 3 minutes or longer, and preferably 30 minutes or shorter, more preferably 20 minutes or shorter.

<50> The method for dyeing hair according to any one of <1> to <49>, wherein a temperature in performing the step (II) is preferably 40°C or lower, more preferably 35°C or lower, and preferably 25°C or higher, more preferably 30°C or higher.

<51> The method for dyeing hair according to any one of <1> to <50>, further comprising, after the step (II), the step of rinsing the hair dye composition applied to hair, wherein the rinsing step is preferably performed by washing off the hair dye composition with water, and more preferably a temperature of the water is from 35 to 45°C.

<52> The method for dyeing hair according to <51>, further comprising, after the hair rinsing step, a drying step of drying the hair, wherein preferably one or more drying treatments selected from the group consisting of towel drying, blow (cold air or hot air) drying, and air drying is performed, and more preferably two or more of the drying treatments are performed in combination.

<53> The method for dyeing hair according to any one of <1> to <52>, wherein the dyeing method comprises no step of applying an oxidizing agent to hair and preferably comprises no step of applying a solution or a composition containing 0.01% by mass or more of the oxidizing agent to hair.

<54> The method for dyeing hair according to any one of <1> to <53>, wherein a difference $\Delta C_A$ between a chroma C value of hair treated by hair dyeing with only the A part and a chroma C value of hair treated by hair dyeing with the hair dye composition, and a difference $\Delta C_B$ between a chroma C value of hair treated by hair dyeing with only the B part and

the chroma C value of hair treated by hair dyeing with the hair dye composition is preferably $\Delta C_A$ of from 1.0 to 12.0 and $\Delta C_B$ of from 1.7 to 12.0, more preferably $\Delta C_A$ of from 2.5 to 11.0 and $\Delta C_B$ of from 2.0 to 10.0, further more preferably $\Delta C_A$ of from 5.0 to 9.0 and $\Delta C_B$ of from 4.0 to 7.5.

<55> A kit for dyeing hair, comprising:

an A part comprising the following component (A1) and component (A2):

(A1) a compound of the following formula (1) or a salt thereof:

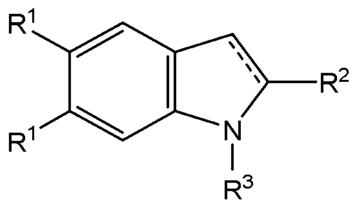

(1)

wherein the broken line represents presence or absence of a $\pi$ bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and

(A2) an alkali agent; and

a B part comprising the following component (B1) and component (B2):

(B1) an oxidation dye other than the component (A1), and

(B2) an alkali agent,

the kit comprising no preparation containing an oxidizing agent.

<56> The kit for dyeing hair according to <55>, wherein the A part has a configuration according to any one of <2> to <6>, <12> to <21>, <26> to <33>, and <36> to <39>.

<57> The kit for dyeing hair according to <55> or <56>, wherein the B part has a configuration according to any one of <7> to <17>, <22> to <31>, and <34> to <39>.

<58> The kit for dyeing hair according to any one of <55> to <57>, wherein the kit is in a two-part form in which the A part and the B part are individually enclosed in their respective containers.

Examples

Hereinafter, Examples of the present invention will be described. However, the present invention is not limited by the scope of Examples. In the present Examples, various measurements and evaluations were performed by the following methods.

<pH measurement>

pH at 25°C of an A part, a B part, and a hair dye composition was measured with a pH meter (F-51, manufactured by HORIBA, Ltd.).

<Feel evaluation>

A hair dyeing treatment was performed in accordance with a method of Example 1 or Comparative Example 1, and a dyed hair tress obtained by drying was rinsed with water. Finger combability during rinsing was evaluated by five expert panelists. A score was determined from the number of panelists who made evaluation as "good finger combability" according to the following criteria:

A: The number of panelists who made evaluation as "good finger combability" was 4 or more.

B: The number of panelists who made evaluation as "good finger combability" was 2 or 3.

C: The number of panelists who made evaluation as "good finger combability" was 1 or less.

<Chroma and difference ΔC after treatment>

A goat hair tress (manufactured by Beaulax Co., Ltd., length: 10 cm, mass: 1.0 g) was used instead of the hair tress. The goat hair tress was dyed by a hair dyeing treatment in accordance with a method of each example. Also, a hair dyeing treatment was performed in each treatment under the same conditions as above except that only the A part or only the B part was used.

Chromaticity coordinates (a* and b*) of the goat hair tress after each hair dyeing treatment were measured at six locations per tress with a colorimeter ("CR-400" manufactured by Konica Minolta, Inc.), and a chroma C* was calculated according to the following equation wherein a* and b* refer to a* and b* defined by the CIE1976 L*a*b* color system.

$$C^* = (a^{*2} + b^{*2})^{1/2}$$

<Difference ΔC>

Subsequently, a difference $\Delta C_A$ of the chroma after the treatment of the present example as to the treatment with only the A part was calculated according to the following equation.

$$\Delta C_A = C^* - C_A^*$$

wherein C* is the chroma of each example, and $C_A^*$ is the chroma from the treatment with only the A part and is a numeric value of Comparative Example 2.

Further, a difference $\Delta C_B$ of the chroma after the treatment of the present example as to the treatment with only the B part was calculated according to the following equation.

$$\Delta C_B = C^* - C_B^*$$

wherein C* is the chroma of each example, and $C_B^*$ is the chroma from the treatment with only the B part and is a numeric value of Comparative Example 3 for Examples 1 and 5 to 10 and Comparative Examples 7 and 8, a numeric value of Comparative Example 4 for Example 2, a numeric value of Comparative Example 5 for Example 3, and a numeric value of Comparative Example 6 for Example 4.

$\Delta C_A$ and $\Delta C_B$ that fall within their respective numeric ranges mentioned above, for example, $\Delta C_A$ of from 1.0 to 12.0 and $\Delta C_B$ of from 1.7 to 12.0, mean good hair dyeability and larger color change than that by the treatment with only the A part or the treatment with only the B part, and enable various colors to be expressed.

<Color difference ΔE between before and after treatment>

A goat hair tress (manufactured by Beaulax Co., Ltd., length: 10 cm, mass: 1.0 g) was used. The goat hair tress was dyed by a hair dyeing treatment in accordance with a method of each example.

The colors of the goat hair tress before the hair dyeing treatment and the goat hair tress after the hair dyeing treatment were measured at six locations per tress with a colorimeter ("CR-400" manufactured by Konica Minolta, Inc.). In this context,

$L_0^*$, $a_0^*$, and $b_0^*$: the color of the goat hair tress before the hair dyeing treatment (average value from measurement at six points)
$L_1^*$, $a_1^*$, and $b_1^*$: the color of the goat hair tress after the hair dyeing treatment (average value from measurement at six points)
In this context, L*, a*, and b* refer to L*, a*, and b* defined by the CIE1976 L*a*b* color system.

A color difference ΔE was determined according to the equation given below. ΔE of 15 or more was evaluated as A, and ΔE of less than 15 was evaluated as C, both of which are shown in the tables. A larger value of ΔE means a higher degree of dyeing.

$$\Delta E = \{(\Delta L^*)^2 + (\Delta a^*)^2 + (\Delta b^*)^2\}^{1/2}$$

$\Delta L^*$: $L_1^* - L_0^*$, $\Delta a^*$:$a_1^* - a_0^*$, and $\Delta b^*$: $b_1^* - b_0^*$.

Example 1 (Hair dyeing and evaluation)

<Preparing tress for evaluation>

An untreated hair tress (black hair of the human head, manufactured by Beaulax Co., Ltd., length: 30 cm, mass: 10 g) was cleaned twice with a plain shampoo whose composition is given below, blow-dried with hot air of a dryer for 30 minutes, and left to stand overnight or longer at a temperature of 25°C under a relative humidity of 65% to prepare a hair tress for the feel evaluation.

An untreated goat hair tress (manufactured by Beaulax Co., Ltd., length: 10 cm, mass: 1.0 g) was treated in the same manner as above to prepare a goat hair tress for the chroma evaluation and the color difference evaluation.

| (Plain shampoo) | (% by mass) |
|---|---|
| ▪ Sodium polyoxyethylene lauryl ether sulfate (EMAL E-27C (amount of active ingredient: 27% by mass), manufactured by Kao Corp.) | 57.4 |
| ▪ Lauramide DEA (AMINON L-02, manufactured by Kao Corp.) | 1.5 |
| ▪ EDTA-2Na (FROST DS, manufactured by Daiichi Pure Chemicals Co., Ltd.) | 0.3 |
| ▪ Phosphoric acid (for pH adjustment to 7.0) | |
| ▪ Sodium benzoate | 0.5 |
| ▪ Purified water | balance |
| Total | 100 |

<Preparing A part and B part>

A component (A1) 5,6-dihydroxyindole and an antioxidant sodium sulfite were blended in the amounts shown in Table 1 with and dissolved in an aqueous solution containing 3% by mass of monoethanolamine in a nitrogen atmosphere. Further, pH was adjusted to 10.2 by adding 75% phosphoric acid as a pH adjuster to prepare an A part.

A component (B1) p-aminophenol and an antioxidant sodium sulfite were blended in the amounts shown in Table 1 with and dissolved in an aqueous solution containing 3% by mass of monoethanolamine in atmosphere. Further, pH was adjusted to 10.2 by adding 75% phosphoric acid as a pH adjuster to prepare a B part.

In the description below, all the amounts of components blended described in the tables are % by mass of the amount of an active ingredient except for phosphoric acid.

The prepared A part was stored in a nitrogen atmosphere, and an aliquot was separated therefrom upon application in each case.

The A part and the B part were mixed such that a molar ratio [(A1)/(B1)] is 50/50 to prepare a hair dye composition (step (I)). Subsequently, the tress for evaluation was rapidly coated with the hair dye composition thus prepared at a bath ratio [dry mass of the tress : mass of the hair dye composition] of 1 : 1 in atmosphere (step (II)) and left to stand in atmosphere for 10 minutes in this state to perform a hair dyeing treatment.

After a lapse of 10 minutes, the tress was washed with running warm water (approximately 40°C) for 30 seconds and then subjected to a cleansing treatment with the plain shampoo described above. The cleansed tress was towel-dried and subsequently blow-dried with cold air of a dryer for 30 minutes. Table 1 shows results of performing feel evaluation in accordance with the method described above with the tress after the drying treatment. Table 2 shows results of performing chroma evaluation after the treatment and color difference evaluation between before and after the treatment.

Comparative Example 1

The A part, the B part, and a C part composed of 5.7% by mass of hydrogen peroxide water were mixed at the ratio described in Table 1 to prepare a hair dye composition containing the oxidizing agent. A hair dyeing treatment and feel evaluation were performed in the same manner as in Example 1 except that the composition was used in the hair dyeing treatment.

[Table 1]

Table 1

| (% by mass) | | | Example 1 | Comparative Example 1 |
|---|---|---|---|---|
| A part | (A1) | Mixture of 5,6-dihydroxyindole and 5,6-dihydroxyindole-2-carboxylic acid | 0.25 | 0.5 |
| | (A2) | Monoethanolamine | 3 | 6 |
| | Antioxidant | Sodium sulfite | 0.4 | 0.4 |
| | pH adjuster | 75% phosphoric acid | q.s. | q.s. |
| | Water | | balance | balance |
| | Total | | 100 | 100 |
| | pH (25°C) | | 10.2 | 10.2 |
| B part | (B1) | p-Aminophenol | 0.183 | 0.366 |
| | (B2) | Monoethanolamine | 3 | 6 |
| | Antioxidant | Sodium sulfite | 0.4 | 0.4 |
| | pH adjuster | 75% phosphoric acid | q.s. | q.s. |
| | Water | | balance | balance |
| | Total | | 100 | 100 |
| | pH (25°C) | | 10.2 | 10.2 |
| C part | Hydrogen peroxide | | - | 5.7 |
| | Purified water | | - | 94.3 |
| | Total | | - | 100 |
| Hair dye composition | pH (25°C) | | 10.2 | 10.2 |
| | Mix ratio (Mass ratio of A part : B part : C part) | | 1 : 1 : 0 | 1 : 1 : 2 |
| | Molar ratio [(A1)/(B1)] | | 50/50 | 50/50 |
| Evaluation result | Feel evaluation | | A | C |

Table 1 shows that the hair dyeing method of Comparative Example 1 including a step of applying an oxidizing agent to hair caused damage after the hair dyeing treatment, presumably resulting in impaired hair feel impression of the hair thus treated. By contrast, as is evident from the table, the hair dyeing method of Example 1 offered favorable feel of hair after the hair dyeing treatment and also had a high chroma and degree of drying after the treatment, because of sufficiently low hair damage as compared with Comparative Example 1 including the step of applying an oxidizing agent.

Examples 2 to 10 and Comparative Examples 2 to 8

A hair dyeing treatment, chroma evaluation after the treatment, and color difference evaluation between before and after the treatment were performed in the same manner as in Example 1 except that the blends of the A part and the B part, and their mix ratio were varied as shown in Table 2 or 3. The results are shown in Table 2 or 3.

Comparative Examples 2 to 6

An A part and a B part each having the composition shown in Table 2 were prepared and mixed at a mass ratio of 1 : 1 to prepare a hair dye composition. A hair dyeing treatment, chroma evaluation after the treatment, and color difference evaluation between before and after the treatment were performed in the same manner as in Example 1 using the composition. The results are shown in Table 2.

The hair dyeing treatments of Examples 2 to 10 and Comparative Examples 2 to 8 included no step of applying an oxidizing agent to the tress, so that feel evaluation was omitted on the assumption that damage on the tress would be sufficiently low, as in Example 1.

[Table 2]

Table 2

| (% by mass) | | | Example 1 | Example 2 | Example 3 | Example 4 | Comparative Example 2 | Comparative Example 3 | Comparative Example 4 | Comparative Example 5 | Comparative Example 6 |
|---|---|---|---|---|---|---|---|---|---|---|---|
| A part | (A1) | Mixture of 5,6-Dihydroxyindole and 5,6-dihydroxyindole-2-carboxylic acid | 0.25 | 0.25 | 0.25 | 0.25 | 0.25 | - | - | - | - |
| | (A2) | Monoethanolamine | 3 | 3 | 3 | 3 | 3 | 3 | 3 | 3 | 3 |
| | Antioxidant | Sodium sulfite | 0.4 | 0.4 | 0.4 | 0.4 | 0.4 | 0.4 | 0.4 | 0.4 | 0.4 |
| | pH adjuster | 75% phosphoric acid | q.s. | q.s. | q.s. | q.s. | q.s. | q.s. | q.s. | q.s. | q.s. |
| | Water | | balance | balance | balance | balance | balance | balance | balance | balance | balance |
| | Total | | 100 | 100 | 100 | 100 | 100 | 100 | 100 | 100 | 100 |
| | pH (25°C) | | 10.2 | 10.2 | 10.2 | 10.2 | 10.2 | 10.2 | 10.2 | 10.2 | 10.2 |
| B part | (B1) Precursor | p-Aminophenol | 0.183 | - | - | - | - | 0.183 | - | - | - |
| | | Toluene-2,5-diamine | - | 0.205 | - | - | - | - | 0.205 | - | - |
| | | 1-Hydroxyethyl-4,5-diaminopyrazole | - | - | 0.239 | - | - | - | - | 0.239 | - |
| | | 2,5,6-Triamino-4-pyrimidinol | - | - | - | 0.237 | - | - | - | - | 0.237 |
| | (B2) | Monoethanolamine | 3 | 3 | 3 | 3 | 3 | 3 | 3 | 3 | 3 |
| | Antioxidant | Sodium sulfite | 0.4 | 0.4 | 0.4 | 0.4 | 0.4 | 0.4 | 0.4 | 0.4 | 0.4 |
| | pH adjuster | 75% phosphoric acid | q.s. | q.s. | q.s. | q.s. | q.s. | q.s. | q.s. | q.s. | q.s. |
| | Water | | balance | balance | balance | balance | balance | balance | balance | balance | balance |
| | Total | | 100 | 100 | 100 | 100 | 100 | 100 | 100 | 100 | 100 |
| | pH (25°C) | | 10.2 | 10.2 | 10.2 | 10.2 | 10.2 | 10.2 | 10.2 | 10.2 | 10.2 |
| Hair dye composition | pH (25°C) | | 10.2 | 10.2 | 10.2 | 10.2 | 10.2 | 10.2 | 10.2 | 10.2 | 10.2 |
| | Molar ratio [(A1)/(B1)] | | 50/50 | 50/50 | 50/50 | 50/50 | 100/0 | 0/100 | 0/100 | 0/100 | 0/100 |
| Hair dyeing results | C value | | 9.12 | 5.62 | 6.93 | 11.07 | 3.29 | 16.04 | 14.36 | 14.75 | 16.42 |

(continued)

| (% by mass) | | Example 1 | Example 2 | Example 3 | Example 4 | Comparative Example 2 | Comparative Example 3 | Comparative Example 4 | Comparative Example 5 | Comparative Example 6 |
|---|---|---|---|---|---|---|---|---|---|---|
| Evaluation results | Difference in chroma $\Delta C_A$ with respect to only A part | 5.83 | 2.33 | 3.64 | 7.78 | 0 | 12.75 | 11.07 | 11.46 | 13.13 |
| | Difference in chroma $\Delta C_B$ with respect to only B part | 6.92 | 8.74 | 7.82 | 5.35 | 12.75 | 0 | 0 | 0 | 0 |
| | Color difference $\Delta E$ between before and after treatment | A | A | A | A | A | C | C | C | C |

[Table 3]

Table 3

| (% by mass) | | | Example 5 | Example 6 | Example 7 | Example 8 | Example 9 | Example 10 | Comparative Example 7 | Comparative Example 8 |
|---|---|---|---|---|---|---|---|---|---|---|
| A part | (A1) | Mixture of 5,6-dihydroxyindole and 5,6-dihydroxyindole-2-carboxylic acid | 0.25 | 0.25 | 0.25 | 0.25 | 0.25 | 0.25 | 0.25 | 0.25 |
| | (A2) | Monoethanolamine | 3 | 3 | 3 | 3 | 3 | 3 | 3 | 3 |
| | Antioxidant | Sodium sulfite | 0.4 | 0.4 | 0.4 | 0.4 | 0.4 | 0.4 | 0.4 | 0.4 |
| | pH adjuster | 75% phosphoric acid | q.s. | q.s. | q.s. | q.s. | q.s. | q.s. | q.s. | q.s. |
| | Water | | balance | balance | balance | balance | balance | balance | balance | balance |
| | Total | | 100 | 100 | 100 | 100 | 100 | 100 | 100 | 100 |
| | pH (25°C) | | 10.2 | 10.2 | 10.2 | 10.2 | 10.2 | 10.2 | 10.2 | 10.2 |
| B part | (B1) | p-Aminophenol | 0.183 | 0.183 | 0.183 | 0.183 | 0.183 | 0.183 | 0.183 | 0.183 |
| | (B2) | Monoethanolamine | 3 | 3 | 3 | 3 | 3 | 3 | 3 | 3 |
| | Antioxidant | Sodium sulfite | 0.4 | 0.4 | 0.4 | 0.4 | 0.4 | 0.4 | 0.4 | 0.4 |
| | pH adjuster | 75% phosphoric acid | q.s. | q.s. | q.s. | q.s. | q.s. | q.s. | q.s. | q.s. |
| | Water | | balance | balance | balance | balance | balance | balance | balance | balance |
| | Total | | 100 | 100 | 100 | 100 | 100 | 100 | 100 | 100 |
| | pH (25°C) | | 10.2 | 10.2 | 10.2 | 10.2 | 10.2 | 10.2 | 10.2 | 10.2 |
| Hair dye composition | pH (25°C) | | 10.2 | 10.2 | 10.2 | 10.2 | 10.2 | 10.2 | 10.2 | 10.2 |
| | Molar ratio [(A1)/(B1)] | | 70/30 | 60/40 | 40/60 | 30/70 | 20/80 | 10/90 | 80/20 | 5/95 |
| Hair dyeing results | C value | | 4.92 | 6.37 | 9.66 | 11.72 | 13.73 | 15.05 | 2.62 | 15.52 |
| Evaluation results | Difference in chroma $\Delta C_A$ with respect to only A part | | 1.63 | 3.08 | 6.37 | 8.43 | 10.44 | 11.76 | -0.67 | 12.23 |
| | Difference in chroma $\Delta C_B$ with respect to only B part | | 11.12 | 9.67 | 6.38 | 4.32 | 2.31 | 0.99 | 13.42 | 0.52 |
| | Color difference $\Delta E$ between before and after treatment | | A | A | A | A | A | A | A | C |

The following was used as the component (A1) described in the tables.
(A1) Mixture of 5,6-dihydroxyindole and 5,6-dihydroxyindole-2-carboxylic acid: produced by the method described in JP-B-5570161 for use. The molar ratio of the 5,6-dihydroxyindole and the 5,6-dihydroxyindole-2-carboxylic acid was 90 : 10. The component (A1) was prepared as a solution containing 1% by mass thereof in $H_2O$/ethanol = 4/1 (wt/wt), which was blended with A part.

As seen from Tables 2 and 3, the methods of the present Examples have favorable hair dyeability and can easily express various colors.

Industrial Applicability

The present invention provides a method for dyeing hair with a melanin precursor, wherein the method for dyeing hair causes less hair damage ascribable to dyeing, has favorable hair dyeability, and can easily express various colors.

## Claims

1. A method for dyeing hair, comprising the following step (I) and (II) in order:

    (I) mixing an A part with a B part before use to prepare a hair dye composition,

    the A part comprising the following component (A1) and component (A2):

    (A1) a compound of the following formula (1) or a salt thereof:

    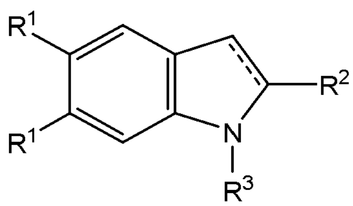 (1)

    wherein the broken line represents presence or absence of a $\pi$ bond; $R^1$ represents a hydroxy group or an acetoxy group; $R^2$ represents a hydrogen atom or -COOR, wherein R represents a hydrogen atom, a methyl group, or an ethyl group; and $R^3$ represents a hydrogen atom, an acetyl group, a methyl group, or an ethyl group, and
    (A2) an alkali agent,

    the B part comprising the following component (B1) and component (B2):

    (B1) an oxidation dye other than the component (A1), and
    (B2) an alkali agent; and

    (II) applying the hair dye composition to hair,
    wherein

    a mix ratio of the A part and the B part in the step (I) is such that a molar ratio of the component (A1) to the component (B1), [(A1)/(B1)], is from 10/90 to 70/30, and
    the method for dyeing hair comprises no step of applying an oxidizing agent to hair.

2. The method for dyeing hair according to claim 1, wherein the mix ratio of the A part and the B part is such that the molar ratio of the component (A1) to the component (B1), [(A1)/(B1)], is from 40/60 to 60/40.

3. The method for dyeing hair according to claim 1 or 2, wherein the component (A2) or the component (B2) comprises one or more members selected from the group consisting of ammonia, alkanolamine, alkylamine, and aralkylamine.

4. The method for dyeing hair according to any one of claims 1 to 3, wherein the component (A2) or the component (B2) comprises one or more members selected from the group consisting of monoethanolamine, trishydroxymethylaminomethane, and 2-amino-2-methyl-1-propanol.

**5.** The method for dyeing hair according to any one of claims 1 to 4, wherein a content of the component (A2) in the A part or a content of the component (B2) in the B part is 0.01% by mass or more and 10% by mass or less.

**6.** The method for dyeing hair according to any one of claims 1 to 5, wherein the A part further comprises an antioxidant as a component (A3).

**7.** The method for dyeing hair according to any one of claims 1 to 6, wherein pH at 25°C of the hair dye composition is 8.0 or higher and 11.0 or lower.

**8.** The method for dyeing hair according to any one of claims 1 to 7, wherein the mixing before use in the step (I) is performed by taking the A part and the B part out of their respective containers where the parts have been prepared, and mixing the parts in a fresh container.

**9.** A kit for dyeing hair, comprising:

an A part comprising the following component (A1) and component (A2):

(A1) a compound of the following formula (1) or a salt thereof:

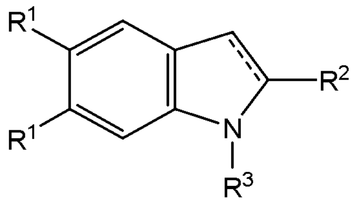

(1)

wherein the broken line represents the presence or absence of a $\pi$ bond; $R^1$ represents a hydroxy group or an acetoxy group; $R^2$ represents a hydrogen atom or -COOR, wherein R represents a hydrogen atom, a methyl group, or an ethyl group; and $R^3$ represents a hydrogen atom, an acetyl group, a methyl group, or an ethyl group, and
(A2) an alkali agent; and

a B part comprising the following component (B1) and component (B2):

(B1) an oxidation dye other than the component (A1), and
(B2) an alkali agent,

the kit comprising no preparation containing an oxidizing agent.

## INTERNATIONAL SEARCH REPORT

International application No.
**PCT/JP2024/002633**

**A. CLASSIFICATION OF SUBJECT MATTER**

***A61K 8/49***(2006.01)i; ***A61K 8/19***(2006.01)i; ***A61K 8/41***(2006.01)i; ***A61Q 5/10***(2006.01)i
FI: A61K8/49; A61K8/41; A61K8/19; A61Q5/10

According to International Patent Classification (IPC) or to both national classification and IPC

**B. FIELDS SEARCHED**

Minimum documentation searched (classification system followed by classification symbols)

A61K8/49; A61K8/19; A61K8/41; A61Q5/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2019-094326 A (KAO CORPORATION) 20 June 2019 (2019-06-20) claims, examples 27, 28 | 1-9 |
| A | JP 5-058860 A (L'OREAL) 09 March 1993 (1993-03-09) entire text, all drawings | 1-9 |
| A | JP 7-508271 A (L'OREAL) 14 September 1995 (1995-09-14) entire text, all drawings | 1-9 |
| A | JP 2019-182829 A (KAO CORPORATION) 24 October 2019 (2019-10-24) entire text, all drawings | 1-9 |
| A | JP 2019-182828 A (KAO CORPORATION) 24 October 2019 (2019-10-24) entire text, all drawings | 1-9 |
| A | JP 2019-182830 A (KAO CORPORATION) 24 October 2019 (2019-10-24) entire text, all drawings | 1-9 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 February 2024** | **12 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/002633**

| Patent document cited in search report | Publication date (day/month/year) | Patent family member(s) | Publication date (day/month/year) |
|---|---|---|---|
| JP 2019-094326 A | 20 June 2019 | US 2020/0397680 A1<br>claims, example 27, 28<br>WO 2019/098365 A1<br>EP 3714870 A1<br>CN 111356437 A | |
| JP 5-058860 A | 09 March 1993 | US 5275626 B1<br>EP 498707 A1 | |
| JP 7-508271 A | 14 September 1995 | US 5538517 B1<br>WO 1994/000100 A1<br>EP 645999 A1 | |
| JP 2019-182829 A | 24 October 2019 | US 2020/0360256 A1<br>WO 2019/098375 A1<br>EP 3714869 A1<br>CN 111372562 A | |
| JP 2019-182828 A | 24 October 2019 | US 2020/0360255 A1<br>WO 2019/098374 A1<br>EP 3714867 A1<br>CN 111372563 A | |
| JP 2019-182830 A | 24 October 2019 | WO 2019/098376 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI358917 A
- JP 3058917 A
- JP HEI1252673 A
- JP 1252673 A
- JP HEI358916 A
- JP 3058916 A
- JP HEI4295414 A
- JP 4295414 A
- CN 114177114
- JP 2001511436 A
- JP 5570161 B

### Non-patent literature cited in the description

- **TERUO HORIUCHI**. *Fragrance Journal*, December 2019, vol. 18 (12), 69-86